(19) **Europäisches Patentamt / European Patent Office / Office européen des brevets**

(11) **EP 2 388 043 B1**

(12) **FASCICULE DE BREVET EUROPEEN**

(45) Date de publication et mention
de la délivrance du brevet:
**23.03.2016 Bulletin 2016/12**

(51) Int Cl.:
*H04W 72/08* (2009.01)    *H04B 17/382* (2015.01)
*A61N 1/372* (2006.01)    *H04B 5/00* (2006.01)

(21) Numéro de dépôt: **11162935.8**

(22) Date de dépôt: **19.04.2011**

(54) **Procédé de recherche et de sélection d'un canal de télémétrie RF pour l'établissement d'une liaison avec un dispositif médical actif**

Such- und Auswahlverfahren eines RF-Telemetriekanals zur Herstellung einer Verbindung mit einer aktiven medizinischen Vorrichtung

Method for searching and selecting an RF telemetry channel to establish a link with an active medical device

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.05.2010 FR 1053858**

(43) Date de publication de la demande:
**23.11.2011 Bulletin 2011/47**

(73) Titulaire: **Sorin CRM SAS**
**92143 Clamart Cedex (FR)**

(72) Inventeur: **Scordilis, Thierry**
**38320 POISAT (FR)**

(74) Mandataire: **Dupuis-Latour, Dominique**
**Bardehle Pagenberg**
**10, boulevard Haussmann**
**75009 Paris (FR)**

(56) Documents cités:
EP-A1- 1 862 195    WO-A1-2007/114743
US-A1- 2008 015 655    US-B1- 6 441 747

**Description**

L'invention concerne les "dispositifs médicaux actifs" tels que définis par la directive 93/42/CE du 14 juin 1993 du Conseil des communautés européennes, et notamment les implants de type "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les pompes de diffusion de substances médicales, les implants cochléaires, les capteurs biologiques implantés, etc., ainsi que les dispositifs de mesure de pH ou encore d'impédance intracorporelle (telle que mesure d'impédance transpulmonaire ou d'impédance intracardiaque). On notera également que, si l'invention s'applique de manière particulièrement avantageuse aux appareils implantés tels que stimulateurs, cardioverteurs ou défibrillateurs, elle peut tout aussi bien être mise en oeuvre avec des dispositifs médicaux non implantés, par exemple des enregistreurs de données comme les appareils Holter externes destinés à la surveillance et à l'enregistrement en ambulatoire de certains paramètres physiologiques tels que par exemple l'activité cardiaque.

La plupart des dispositifs médicaux actifs sont conçus pour permettre un échange de données avec un "programmateur", qui est un appareil externe permettant de vérifier le paramétrage du dispositif, de lire des informations enregistrées par celui-ci ou d'y inscrire des informations, ou encore de mettre à jour le logiciel interne de pilotage du dispositif.

Cet échange de données entre le dispositif médical et le programmateur est effectué par télémétrie, c'est-à-dire par une technique de transmission à distance d'informations, sans contact galvanique.

Jusqu'à présent, la télémétrie était le plus souvent opérée par couplage inductif entre des bobines du dispositif implanté et du programmateur, technique connue sous le nom de "procédé par induction". Cette technique présente cependant l'inconvénient, en raison de la très faible portée d'un tel couplage, de nécessiter l'utilisation d'une "tête de télémétrie" reliée au programmateur et contenant une bobine qu'un opérateur place au voisinage du site où est implanté le dispositif.

Il a été récemment proposé de mettre en oeuvre une autre technique de couplage non galvanique, utilisant les deux composantes d'une onde électromagnétique produite par des circuits émetteurs/récepteurs opérant dans le domaine des radiofréquences (RF), typiquement des fréquences de l'ordre de plusieurs centaines de mégahertz.

Cette technique, dite de "télémétrie RF" permet de programmer ou interroger des implants à des distances supérieures à 3 m, et autorise donc l'échange d'informations sans manipulation d'une tête de télémétrie, voire même sans intervention d'un opérateur externe.

Un dispositif médical actif comprenant de tels moyens de télémétrie RF est par exemple décrit dans le EP 1 862 195 A1 (ELA Medical).

Le protocole de communication entre le dispositif actif (généralement un implant) et la station de base (programmateur du dispositif de "home monitor") est notamment régi par la norme EN 301839 *"Compatibilité électromagnétique et spectre radioélectrique (ERM) - Dispositifs à courte portée (SRD) - Implantes médicaux actifs de puissance ultra basse (ULP-AMI) et périphériques (ULP-AMI-P) fonctionnant dans la plage de fréquences de 402 MHz à 405 MHz"*. On notera toutefois que l'invention n'est pas limitée à une utilisation dans la bande 402-405 MHz, dite bande MICS *(Medical Implants Communication Systems),* mais qu'elle est applicable de façon générale à toutes les bandes susceptibles d'être utilisées pour de la télémétrie RF, notamment les bandes banalisées publiques ISM (*Industriel, Scientifique et Médical*) 863-870 MHz, 902-928 MHz et 2,4 GHz utilisées par les dispositifs médicaux.

Par ailleurs, les appareils médicaux dotés de fonctions de télémétrie RF sont tous des appareils multicanaux, c'est-à-dire utilisant plusieurs fréquences situées dans une même bande (certains appareils pouvant être en outre multibande, mais il s'agit là d'un aspect qui ne concerne pas l'invention).

De ce fait, préalablement à l'établissement d'une communication entre l'implant et la station de base, il est nécessaire de sélectionner un canal (c'est-à-dire une fréquence sur laquelle la communication sera opérée) parmi tous les canaux susceptibles d'être utilisés dans la bande considérée.

Cette étape est importante, car il est indispensable de choisir un canal peu bruité, sur lequel on est pratiquement sûr que la communication pourra être conduite jusqu'à son terme sans être interrompue.

En effet, à la différence des techniques par induction qui présentent une bonne immunité aux parasites, la télémétrie RF est sujette à de nombreuses perturbations par l'environnement électromagnétique, notamment les signaux de radio, de télévision et de téléphonie mobile, sans compter les nombreux parasites industriels susceptibles d'être produits dans l'environnement immédiat du porteur de l'implant. L'implant qui veut communiquer sur un canal RF peut également se trouver en conflit avec d'autres dispositifs en train de communiquer situés à proximité qui, eux aussi, occupent des canaux de télémétrie RF. Toutes ces perturbations sont susceptibles de produire des interférences et de perturber la transmission des données.

Si l'on a sélectionné un canal trop bruité, la communication sera sujette à de nombreuses erreurs de transmission, conduisant à l'abandon du processus et à la recherche d'un nouveau canal. Il sera de ce fait nécessaire de réitérer complètement le processus de communication, et on aura dépensé de l'énergie pour une communication qui n'a pas abouti, donc pour rien.

Or la télémétrie RF implique une dépense énergétique relativement importante, tout au moins à l'échelle d'un implant dont la durée de vie est un paramètre extrêmement critique, de sorte que la multiplication des communications interrompues pourra avoir à terme une incidence non négligeable sur l'autonomie de l'implant.

Pour sélectionner un canal, on procède généralement par une scrutation préalable consistant à "écouter" successivement les différents canaux susceptibles d'être utilisés pour l'émission, avant de choisir l'un d'entre eux pour commencer à émettre un signal.

Le US 6 868 288 B2 (US 2002/0045920 A1) propose, pour opérer ce choix préalable du canal de communication, d'analyser simultanément le niveau moyen de signal capté sur chacun des canaux, puis de classer ceux-ci en fonction du niveau détecté respectif (tout signal présent sur un canal étant considéré comme du bruit). Le canal retenu sera ensuite analysé isolément pour vérifier qu'il est bien disponible au moment où le dispositif va commencer à émettre : en effet, entre le moment de la scrutation et celui du début de l'émission, une communication aura peut-être commencé sur ce canal en provenance d'un autre dispositif, de sorte que le canal devenu occupé ne sera plus disponible pour l'émission.

La technique décrite par ce document présente deux inconvénients principaux.

En premier lieu, du fait du critère d'analyse choisi, tout signal présent dans le canal est considéré comme du bruit. En d'autres termes, l'analyse ne fait aucune distinction entre du bruit de fond (c'est-à-dire du "vrai" bruit) et la présence d'un signal utile du fait d'une communication en cours sur ce canal : cette dernière est assimilé par le dispositif à du bruit, alors que cette émission peut très bien avoir lieu sur un canal de bonne qualité du point de vue du rapport signal/bruit. En d'autres termes, le dispositif peut très bien exclure un canal peu bruité, du seul fait que ce canal se trouve occupé à l'instant de la scrutation.

En second lieu, l'analyse du seul niveau moyen de signal sur un canal au moment de la scrutation ne garantit pas que le canal sera effectivement libre au moment où l'on souhaitera commencer à émettre sur ce canal. Le risque évoqué plus haut subsiste, à savoir le risque d'une impossibilité d'établir la communication et la nécessité de passer à un autre canal en réitérant le processus, avec pour conséquence une inutile dépense d'énergie.

On notera que ce risque est particulièrement aggravé dans un milieu hospitalier où peuvent être simultanément présents plusieurs dispositifs d'un même fabricant (donc utilisant les mêmes algorithmes de choix de canal). En effet, il peut se trouver que plusieurs appareils veuillent établir une communication RF à des instants relativement proches et, du fait de ce léger décalage temporel, un dispositif pourra considérer qu'un canal est libre, alors que l'autre commencera à émettre peu de temps après et rendra impossible la communication du premier, obligeant ce dernier à réitérer la scrutation pour rechercher un autre canal, etc. La présence dans un même site d'une pluralité de produits de même origine peut être donc une source imprévue de brouillage, nécessitant de prévoir des logiques de fonctionnement avec des boucles d'échappatoire assez complexes.

Ce phénomène est en outre accru par l'occupation globale relativement élevée des différents canaux en milieu clinique, le problème de trouver un canal disponible se surajoutant à celui de trouver un canal peu parasité. Le US2008/015655 A1 (Bange et al.) décrit une technique comparable mais présentant les mêmes inconvénients, à savoir l'impossibilité d'anticiper le risque qu'un canal apparemment peu bruité à un instant donné soit réellement le canal optimal au moment où ce canal sera utilisé, et ce pendant toute la durée de son utilisation.

Il serait de ce fait souhaitable de disposer d'une technique de recherche et de sélection de canal qui permette de distinguer entre un canal occupé et un canal présentant un niveau significatif de bruit parasite manière à pouvoir identifier des canaux présentant un bon rapport signal/bruit, même si ceux-ci sont occupés, afin de pouvoir les utiliser ultérieurement lorsqu'il seront libres.

Le but de l'invention est de pallier les divers inconvénients exposés ci-dessus, en proposant une technique d'analyse et de sélection des canaux permettant de maximiser la probabilité d'établir une connexion sur ce canal et de poursuivre celle-ci jusqu'à son terme.

En d'autres termes, le but de l'invention est de proposer une nouvelle technique de sélection non seulement d'un canal libre et peu bruité au moment de la scrutation, mais d'un canal qui ait des chances de rester libre au moment où le dispositif tentera d'établir la communication.

Il s'agit ainsi de maximiser la probabilité d'établir et de mener à son terme une communication sur ce canal dès la première tentative, en diminuant par conséquent la quantité d'énergie globale nécessaire pour transférer des informations entre l'implant et la station de base, avec pour conséquence un impact minimum de la télémétrie RF sur la durée de vie de cet implant.

Un autre but de l'invention est de proposer une telle technique qui n'exige que des ressources modestes en termes de puissance de calcul, ne faisant intervenir que des algorithmes simples, ou même des logiques susceptibles d'être implémentées sous forme matérielle à partir de comparateurs et de circuits à seuil, d'une façon particulièrement avantageuse en ce qui concerne l'implant.

L'invention propose à cet effet un procédé de recherche et de sélection d'un canal de communication préalablement à l'établissement d'une liaison de télémétrie RF avec un dispositif distant, ce procédé étant du type général connu d'après le US2008/015655 A1 précité, correspondant au préambule de la revendication 1. La partie caractérisante énonce les éléments particuliers de l'invention.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 est une vue schématique des différents blocs fonctionnels du circuit de réception d'un dispositif médical mettant en oeuvre l'invention.

La Figure 2 est un organigramme général expliquant les différentes étapes de l'analyse à long terme, en dehors des périodes de communication, et à court terme, en cas de demande de communication, et la combinaison des résultats obtenus pour la détermination des critères de sélection du canal.

La Figure 3 est un organigramme illustrant de manière plus précise la manière dont sont opérée les analyses à long terme et à court terme de l'organigramme de la Figure 2.

Les Figures 4a et 4b illustrent des exemples de signaux analysés et discriminés par le classifieur de l'invention.

La Figure 5 est un exemple de signaux qu'il est possible de mesurer sur dix canaux successifs, montrant divers niveaux de signal et divers types de brouillage.

La Figure 6 illustre de façon schématique la manière dont l'invention est mise en oeuvre, avec les phases successives d'analyse à long terme, d'analyse à court terme, et de sélection du canal en fonction du résultat de ces deux analyses.

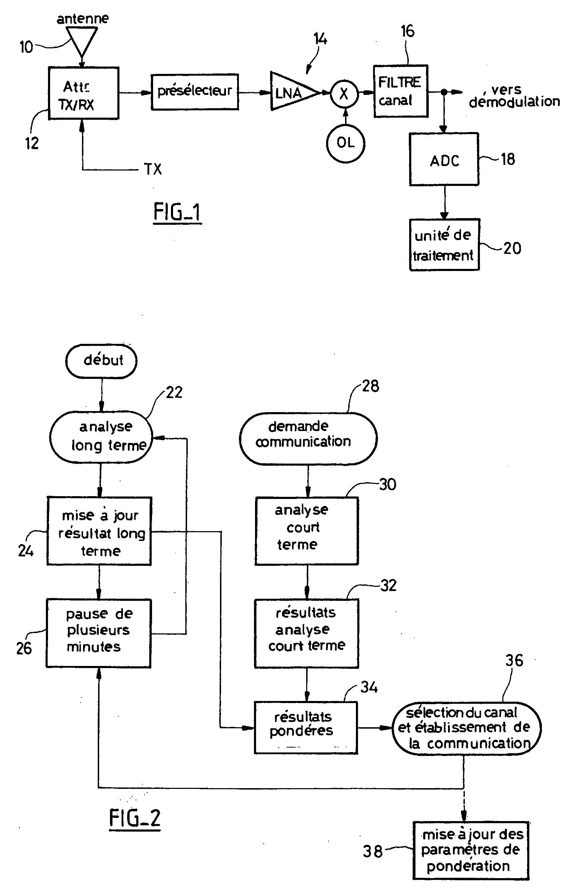

Sur la Figure 1, on a représenté de façon schématique, sous forme de blocs fonctionnels, les principaux circuits de l'étage de réception d'un dispositif médical (implant ou station de base).

Les signaux captés par une antenne RF 10 sont appliqués à un étage atténuateur 12 commandé par un signal TX. Concrètement, au cours d'une communication déjà établie, l'atténuateur 12 est en position "RX" de réception, c'est-à-dire d'atténuation minimale. En revanche, dans une phase préalable de scrutation des canaux, lorsque l'on n'a pas connaissance du niveau de signal pouvant être présent sur ce canal, l'atténuateur est basculé sur la position "TX" d'émission (et ceci bien que le dispositif continue à opérer en réception) de manière à éviter de saturer les étages situés en aval ; ce n'est que si l'on détecte un signal suffisamment faible que l'atténuateur 12 sera basculé sur la position "RX" pour bénéficier de la sensibilité maximale.

Le signal ainsi recueilli est appliqué ensuite à une série d'étages haute fréquence 14 comprenant un présélecteur (qui permet de ne garder que la bande de fréquences d'intérêt et permet de protéger le récepteur d'autres émissions RF, le média étant un média partagé), un amplificateur faible bruit LNA (pour augmenter le rapport signal/bruit), oscillateur local OL et mélangeur X, avec une architecture superhétérodyne classique de récepteur RF. Le signal à fréquence intermédiaire obtenu est appliqué à un filtre réjecteur 16 correspondant au canal sélectionné (pour ne garder que les signaux présents dans le canal d'intérêt), par exemple un filtre comprenant un résonateur à ondes acoustiques de volume BAW (*Bulk Acoustic Waves*) tel que celui décrit par exemple dans le EP 1 862 195 A1 précité. La configuration que l'on vient de décrire présente l'intérêt de pouvoir figer le circuit, et de ne capter que la fréquence que l'on souhaite, simplement en changeant la fréquence de l'oscillateur local. On s'intéressera au signal issu de ce filtre de canal 16 afin de le numériser et de permettre au processeur central de traiter l'information correspondante, et ce canal par canal.

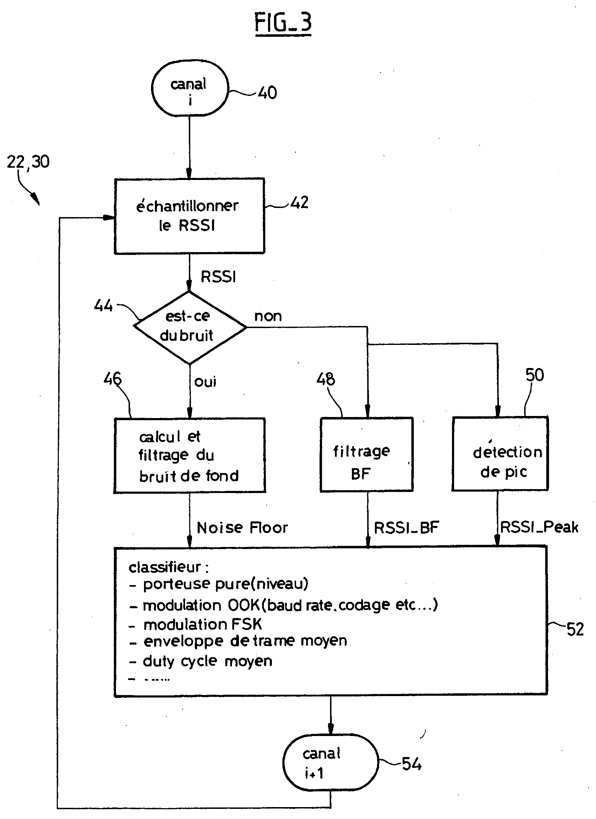

Le signal recueilli en sortie du filtre de canal 16, qui normalement est appliqué au circuit de démodulation dans le cadre d'une communication déjà établie, est ici utilisé pour détecter le niveau de signal après avoir été appliqué à un convertisseur analogique/numérique 18 et à une unité de traitement 20 dont on va décrire le fonctionnement détaillé en référence aux Figures 2 et 3.

La Figure 2 illustre le principe de l'invention, qui consiste à opérer deux types d'analyse, à savoir une "analyse à long terme" avec scrutation des canaux à intervalles réguliers en l'absence de toute demande de communication, et une "analyse à court terme" avec scrutation des canaux au moment précis où survient une demande d'établissement d'une communication.

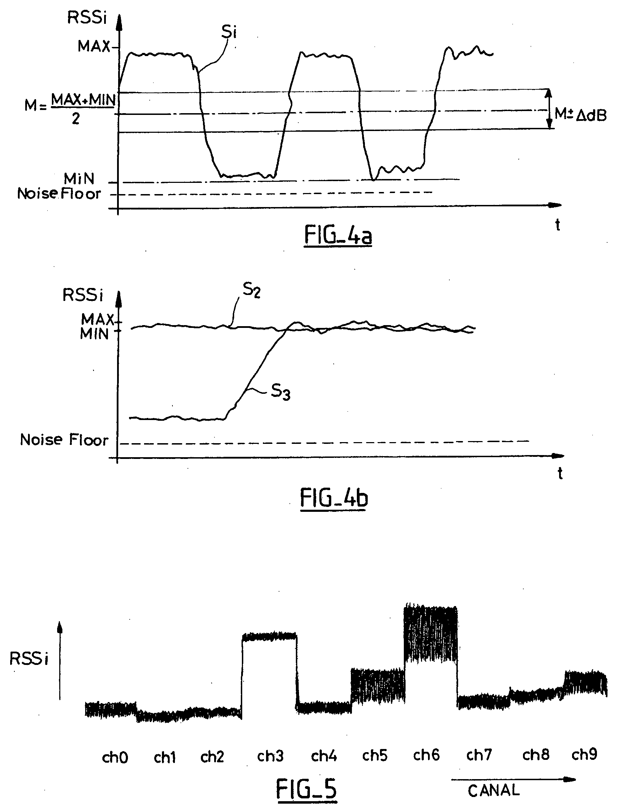

L'analyse à long terme (étape 22), dont le principe détaillé sera exposé en référence à la Figure 5, est opérée à intervalles réguliers, par exemple toutes les quinze minutes.

Cette analyse est effectuée par la station de base car on profite du fait que l'on dispose d'une puissance de calcul disponible et de suffisamment d'énergie pour effectuer l'analyse des canaux depuis cette station de base. Cela étant, l'invention pourrait être également mise en oeuvre *mutatis mutandis* à partir de l'implant, bien qu'une telle mise en oeuvre soit beaucoup plus critique notamment sur le plan de la consommation énergétique.

La majeure partie du temps la station de base est inactive, avec une consommation électrique minimale. À intervalles réguliers, elle se réveille pendant une période active de quelques secondes pour procéder à l'analyse (étape 22) des différents canaux disponibles. Les résultats de cette analyse sont mémorisés et/ou mis à jour par rapport aux résultats antérieurs (étape 24), puis la station de base retourne à un état dormant pendant plusieurs minutes (étape 26), en attendant d'être réveillée pour la prochaine période d'analyse ou sur réception d'une interruption spécifique.

Les étapes 22 et 24 d'analyse et de mémorisation, qui seront détaillées par la suite en référence à la Figure 3, consistent essentiellement à "écouter" successivement les différents canaux et à construire une table avec pour chaque canal un indicateur représentatif de la disponibilité à long terme de celui-ci. L'ensemble de cette table procure ainsi une vue globale du "paysage électromagnétique" au voisinage de la station de base.

Lorsqu'une demande de communication survient (étape 28), par exemple à réception d'un signal émis par l'implant, la station de base va scruter les différents canaux susceptibles d'être utilisés pour la communication, afin de décider du canal qui sera utilisé pour l'échange des signaux, conformément à l'étape LBT (*Listen Before Talk*) du protocole de communication prescrit par la norme EN 301839.

La station de base opère à cet effet une "analyse à court terme" (étape 30) de même nature que l'analyse à long terme (étape 22) qui avait été opérée auparavant en l'absence de demande de communication.

De façon caractéristique de l'invention, les résultats de cette analyse à court terme sont mémorisés (étape 32) et combinés (étape 34) aux résultats obtenus de l'analyse à long terme, de manière à produire des résultats pondérés à partir desquels un canal sera sélectionné et la communication établie sur ce canal (étape 36).

Les paramètres de pondération seront mis à jour (étape 38) à l'issue de la communication, pour tenir compte du fait que celle-ci s'est déroulée correctement jusqu'à son terme ou, au contraire, a dû être interrompue en raison d'erreurs de transmission trop nombreuses.

La Figure 3 illustre la manière dont sont opérées les analyses à court terme ou à long terme des étapes 22 et 30 de la Figure 2.

Pour chaque canal i sélectionné lors de la scrutation (étape 40), le niveau du signal capté en réception est échantillonné (étape 42), ce niveau étant désigné conventionnellement RSSI (*Received Signal Strength Indicator*). Le RSSI est ensuite comparé à un seuil relativement bas (étape 44), de manière à déterminer si le signal capté en réception est suffisamment faible pour être considéré comme du bruit de fond.

Dans l'affirmative, un niveau de bruit de fond est calculé à partir du RSSI (étape 46), donnant une valeur *NoiseFloor* représentative de ce niveau de bruit de fond. On peut notamment calculer une moyenne glissante à grande fenêtre, permettant de filtrer au maximum le signal afin d'en évaluer le bruit de fond. Un nouvel échantillon ne sera introduit dans cette moyenne que s'il apparait comme étant du signal, c'est-à-dire s'il présente une amplitude supérieure à l'échantillon de bruit de fond précédent, selon les relations :

$$\Delta_{i+1} = \frac{Average_i - Data_{i+1}}{window\ Length}$$

$$Average_{i+1} = Average_i + \Delta_{i+1}$$

S'il ressort du test 44 que le signal capté en réception n'est vraisemblablement pas constitué d'un simple bruit de fond, il ne doit pas figurer dans le calcul du niveau de *NoiseFloor* (ce qui reviendrait à augmenter artificiellement le bruit de fond, de manière non réaliste).

Dans ce dernier cas, le signal reçu est soumis à un filtrage basse fréquence (étape 48) pour délivrer un indicateur *RSSI_BF* représentatif du niveau du RSSI hors bruit de fond. Le calcul à cette étape 48 est semblable à celui du bloc 46, sauf qu'ici la fenêtre est choisie pour ne pas trop filtrer le signal pour en extraire la tendance (on s'approche de la fréquence de Shannon sans la réaliser, pour être cohérent avec la fréquence modulante). Le signal est également appliqué à un étage de détection de pic (étape 50) permettant de détecter un pic de niveau apparaissant dans le canal, même de façon temporaire, pour pouvoir prendre en compte notamment un effet de traîne représentatif d'une saturation.

Pour détecter ce pic, on procède de la manière suivante : si le nouvel échantillon est supérieur au précédent, alors on conserve la valeur, dans le cas contraire on diminue simplement la valeur maximale mémorisée. Ceci permet de garder une trace de l'existence de ce pic et, en mesurant le temps pendant lequel ce pic est supérieur à la moyenne du bloc 50, on peut en estimer l'amplitude crête et mesurer des fréquences de répétition afin de savoir si l'on est en présence d'un phénomène périodique ou non. La détection de pic du bloc 50 délivre un indicateur *RSSI_Peak* représentatif de l'énergie du pic éventuel. De façon générale, on aura *RSSI_Peak > RSSI_BF > NoiseFloor.*

Les trois paramètres *NoiseFloor, RSSI_BF* et *RSSI_Peak* sont ensuite combinés (étape 52) de manière à opérer une classification permettant de discriminer le type de signal capté en réception : bruit de fond uniquement, porteuse pure, porteuse modulée (avec différents types de modulation), etc.

Cette discrimination sera expliquée en référence aux Figures 4a et 4b, qui illustrent des exemples de signaux analysés et discriminés par le classifieur de l'invention. d'une succession de créneaux. On a également représenté le niveau maximal MAX, le niveau minimal MIN et le niveau moyen M (M = (MAX+MIN)/2) de ce signal. On a en outre défini un intervalle M $\pm$ $\Delta$, par exemple un intervalle de $\pm$ 3 dB, autour du niveau M.

Le classifieur considérera que le signal capté est effectivement un signal modulé en amplitude si les conditions suivantes sont vérifiées :

- le niveau maximum MAX du signal est supérieur d'au moins 3dB (par exemple) au niveau *NoiseFloor* du bruit de fond ;
- le nombre d'échantillons du signal capté numérisé qui se situent au niveau maximum MAX correspond (à un facteur d'incertitude près) au nombre d'échantillons qui se situent au niveau minimum MIN, ce qui revient à dire que les échantillons de signal sont équirépartis entre le maximum et le minimum du signal ; et
- la moyenne des niveaux des échantillons est située dans l'intervalle M $\pm$ $\Delta$ autour du niveau M = (MAX+MIN)/2.

Sur la Figure 4b on a représenté en $S_2$ un signal brut reçu qui est une porteuse pure ou bien un signal modulé en fréquence.

Le classifieur considérera que le signal capté est effectivement un tel signal si les conditions suivantes sont vérifiées :

- le niveau maximum MAX du signal est supérieur d'au moins 3dB (par exemple) au niveau *NoiseFloor* du bruit de fond ;
- le nombre d'échantillons du signal capté numérisé qui se situent au niveau maximum MAX correspond (à un facteur d'incertitude près) au nombre d'échantillons qui se situent au niveau minimum MIN, ce qui revient à dire que les échantillons de signal sont équirépartis entre le maximum et le minimum du signal ;
- la différence entre les niveaux MAX et MIN du signal est inférieure à un seuil limite, par exemple cette différence est inférieure à 3 dB.

Une discrimination supplémentaire peut être opérée pour voir si le signal est en cours d'apparition (signal $S_3$) ou non (signal $S_2$), en mesurant le "taux de remplissage" qui représente le nombre d'échantillons dont le niveau est proche du maximum par rapport au nombre total d'échantillons. Une fois que l'analyse du signal présent sur le canal *i* a été opérée de cette manière, la même analyse est ensuite effectuée pour le canal suivant *i*+1 (étape 54), et ce jusqu'à ce que tous les canaux aient été explorés.

Dans un fonctionnement normal du dispositif, le paramètre *NoiseFloor,* qui représente le bruit de fond global des circuits électroniques plus le bruit de fond électromagnétique ambiant, ne doit pas varier brutalement. Une variation brutale proviendrait d'un événement non physique, par exemple dû à une détérioration de l'électronique, à des problèmes liés à l'installation ou à une dégradation du produit en fin de vie par exemple. L'analyse des variations de cette valeur *NoiseFloor* peut donc permettre de générer des alertes éventuelles.

La comparaison des paramètres *RSSI_Peak* et *RSSI_BF* permet d'évaluer la durée moyenne des brouilleurs, afin par exemple de distinguer entre un simple pic ou bien une trame de signal, ou même pour identifier la nature de la modulation : par exemple si *RSSI_Peak* = *RSSI_BF*, on se trouve vraisemblablement en présence d'un signal modulé FSK (*Frequency Shift Keying*, modulation à saut de fréquences discrètes) tandis que si *RSSI_Peak* > *RSSI_BF* + $\Delta$, on a vraisemblablement affaire à un signal OOK (*On-Off Keying*, modulation en tout-ou-rien), etc.

On voit ainsi que par des comparaisons relativement simples, pouvant même être réalisées sous forme matérielle, il est possible de reconnaître certains types de brouilleurs dont les caractéristiques auront été identifiées ou apprises, et d'opérer la classification recherchée par un tri des paramètres mesurés.

Le résultat est un indicateur représentatif de la disponibilité du canal, défini par exemple sous forme d'un pourcentage ou d'un taux de confiance, à court terme ou à long terme selon le moment auquel aura été opérée l'analyse.

La Figure 5 illustre un exemple de divers signaux mesurés sur dix canaux *ch0* à *ch9*. On peut notamment remarquer la présence sur le canal *ch3* d'un brouilleur permanent constitué par une porteuse pure, et la présence sur le canal *ch6* d'un brouilleur modulé OOK qui déborde également sur le canal *ch5*.

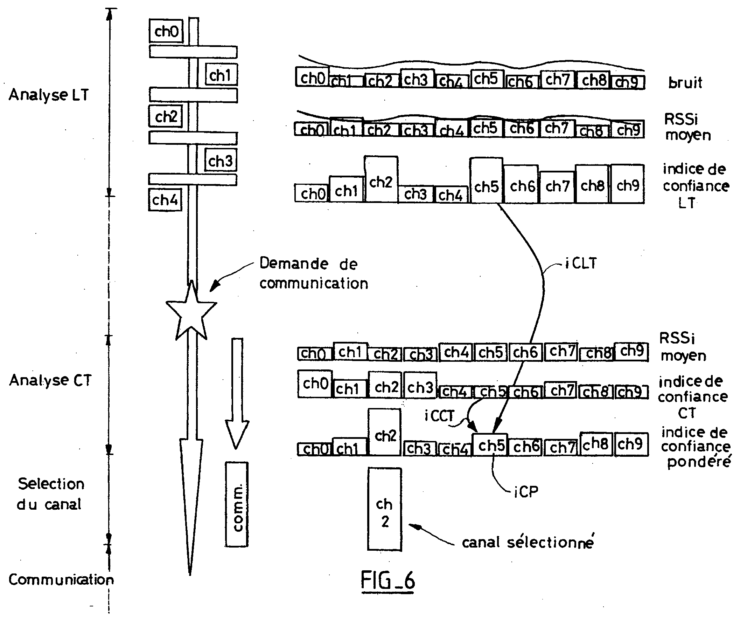

La Figure 6 illustre le déroulement général du processus de l'invention, avec successivement :

- l'analyse à long terme (étape qui est en fait réitérée à intervalles réguliers pendant les périodes d'inactivité de la station de base) ;
- la demande de communication ;
- l'analyse à court terme des canaux ;
- la sélection du canal ; et
- l'établissement et la poursuite de la communication.

Les analyses à long terme et à court terme permettent, pour chacun des canaux *ch0* à *ch9,* d'analyser le bruit, le RSSI moyen, et d'établir un indice de confiance, respectivement à long terme et à court terme.

C'est la combinaison des indices de confiance à long terme (LT) et à court terme (CT), schématisée par les flèches référencées ICLT et ICCT, qui permettent de disposer d'un indice de confiance pondéré ICP à partir duquel sera sélectionné l'un des dix canaux.

Cette combinaison peut être opéré par le biais d'une moyenne glissante, avec des fenêtres de longueurs différentes pour l'analyse court terme et pour l'analyse long terme. Ainsi, pour le canal *chx* d'indice *x* on aura respectivement :

$$moyenne\_glissante\_chx(t+1) = moyenne\_glissante\_chx(t) + (moyenne\_glissante\_chx(t) - chx\_CT) \, / \, fenêtre\_CT \,, \text{ et}$$

$$moyenne\_glissante\_chx(t+1) = moyenne\_glissante\_chx(t) + (moyenne\_glissante\_chx(t) - chx\_LT) \, / \, fenêtre\_LT$$

On peut avoir dans ce cas *fenêtre_LT = N x fenêtre_CT*. N permet alors d'ajuster directement le rapport entre les deux analyses. N peut être fixé arbitrairement comme étant le ratio temporel entre les deux. Par exemple, si l'analyse à court terme est faite une fois par jour alors que l'analyse à long terme est faite une fois par heure, on aura : *N* = 24.

Dans l'exemple illustré Figure 6, c'est le canal *ch2* qui sera finalement sélectionné, car c'est celui qui présente l'indice de confiance pondéré le plus élevé. Certes d'autres canaux, par exemple les canaux *ch0* et *ch3,* présentent un indice de confiance à court terme au moins aussi élevé ; mais l'analyse à long terme a montré que ces canaux *ch0* et *ch3* présentaient un indice de confiance à long terme moindre que celui du canal *ch2,* de sorte qu'ils seront éliminés lors de la sélection du canal préalablement à l'établissement de la communication.

## Revendications

**1.** Un procédé de recherche et de sélection d'un canal de communication parmi une pluralité de canaux, ce procédé étant mis en oeuvre par un dispositif médical actif tel qu'un implant actif ou un programmateur pour un tel implant, préalablement à l'établissement d'une liaison de télémétrie RF avec un dispositif distant, comprenant les étapes suivantes :

- en l'absence de requête de communication ou de communication en cours avec le dispositif distant, scrutation périodique et première analyse (22) de ladite pluralité de canaux avec, pour chaque canal, détermination et mémorisation d'au moins un indicateur long terme (ICLT) représentatif de la disponibilité à long terme du canal ;
- sur réception d'une requête de communication avec le dispositif distant :

• seconde analyse (30) de ladite pluralité de canaux avec, pour chaque canal, détermination et mémorisation d'au moins un indicateur court terme (ICCT) représentatif de la disponibilité à court terme du canal ;
• pondération (34) de l'indicateur court terme par l'indicateur long terme mémorisé, pour donner un indicateur court terme pondéré et
• sélection (36), parmi la pluralité de canaux, d'un canal comme canal de communication sur la base de l'indicateur court terme pondéré,

**caractérisé en ce que** ladite première ou seconde analyse (22, 30) comprend une étape de :

- comparaison (44) du niveau du signal (RSSI) de chaque canal à un seuil prédéterminé de niveau de bruit de base ; et

pour chaque canal dont le niveau de signal est supérieur au niveau de bruit de base, une étape (48, 50, 52) de :

- discrimination de type de signal, apte à différencier un signal utile de trame de données et un signal parasite.

**2.** Le procédé de la revendication 1, dans lequel l'étape de discrimination comprend :

- l'application au signal d'un filtrage passe-bas (48) sur une fenêtre temporelle de durée prédéterminée, et
- la comparaison entre le signal filtré (RSSI_BF) et le signal non filtré (RSSI), pour donner un premier indicateur de type de signal.

**3.** Le procédé de la revendication 1, dans lequel l'étape de discrimination comprend en outre :

- la détection (50) d'un niveau de pic du signal, et
- la comparaison entre le niveau de pic du signal (RSSI_PEAK) et le signal filtré (RSSI_BF), pour donner un deuxième indicateur de type de signal.

## Patentansprüche

1. Such- und Auswahlverfahren eines Kommunikationskanals unter mehreren Kanälen, wobei dieses Verfahren durch eine aktive medizinische Vorrichtung, wie ein aktives Implantat oder ein Programmiergerät für ein solches Implantat, vor der Herstellung einer RF-Telemetrieverbindung mit einer Remote-Vorrichtung umgesetzt wird, das die folgenden Schritte aufweist:

   - in Abwesenheit von einer Kommunikationsanforderung oder einer laufenden Kommunikation mit der Remote-Vorrichtung, periodisches Abtasten und erste Analyse (22) der mehreren Kanäle mit Bestimmen und Speichern für jeden Kanal von mindestens einem Langfrist-Indikator (ICLT), der für die langfristige Verfügbarkeit des Kanals repräsentativ ist;
   - nach Empfang einer Kommunikationsanforderung mit der Remote-Vorrichtung:

     • zweite Analyse (30) der mehreren Kanäle mit Bestimmen und Speichern für jeden Kanal von mindestens einem Kurzfrist-Indikator (ICCT), der für die kurzfristige Verfügbarkeit des Kanals repräsentativ ist;
     • Gewichten (34) des Kurzfrist-Indikators durch den gespeicherten Langfrist-Indikator, um einen gewichteten Kurzfrist-Indikator zu geben und
     • Auswählen (36) aus den mehreren Kanälen eines Kanals als Kommunikationskanal auf der Grundlage des gewichteten Kurzfrist-Indikators,

   dadurch gekennzeichnet, daß die erste oder zweite Analyse (22, 30) eine Schritt des:

   - Vergleichens (44) des Signalpegels (RSSI) jedes Kanals mit einem vorbestimmten Schwellenwert eines Grundgeräuschpegels und

   für jeden Kanal, dessen Signalpegel höher als der Grundgeräuschpegel ist, einen Schritt (48, 50, 52) des:

   - Unterscheidens des Signaltyps aufweist, der geeignet ist, ein Nutzsignal eines Datenrahmens und ein Störsignal zu unterscheiden.

2. Verfahren nach Anspruch 1, wobei der Schritt des Unterscheidens Folgendes aufweist:

   - das Anwenden eines Tiefpaßfilters (48) auf das Signal über ein Zeitfenster von vorbestimmter Dauer und
   - das Vergleichen zwischen dem gefilterten Signal (RSSI_BF) und dem nicht gefilterten Signal (RSSI), um einen ersten Indikator des Signaltyps zu geben.

3. Verfahren nach Anspruch 1, wobei der Schritt des Unterscheidens ferner Folgendes aufweist:

   - das Detektieren (50) eines Spitzenpegels des Signals und
   - das Vergleichen zwischen dem Spitzenpegel des Signals (RSSI_PEAK) und dem gefilterten Signal (RSSI_BF), um einen zweiten Indikator des Signaltyps zu geben.

## Claims

1. A method of search and selection of a communication channel among a plurality of channels, said method being implemented by an active medical device such as an active implant or a programmer for such an implant, previously to the establishment of an RF telemetry link with a remote device, comprising the following steps:

   - in the absence of a request for communication or a communication in progress with the remote device, periodic scanning and first analysis (22) of said plurality of channels with, for each channel, determination and memorization of at least one long term indicator (ICLT) representative of the long term availability of the channel;
   - on receipt of a request for communication with the remote device:

• second analysis (30) of said plurality of channels with, for each channel, determination and memorization of at least one short term indicator (ICCT) representative of the short term availability of the channel;
• weighting (34) of the short term indicator by the long term indicator memorized, to obtain a weighted short term indicator, and
• selection (36) of a channel, among the plurality of channels, as a communication channel on the basis of the weighted short term indicator,

**characterized in that** said first or second analysis (22, 30) comprises a step of:

- comparison (44) of the signal level (RSSI) of each channel to a predetermined threshold of base noise level; and

for each channel whose signal level is higher than the base noise, a step (48, 50, 52) of:

- signal type discrimination, adapted to make the difference between a useful data frame signal and a spurious signal.

2. The method of claim 1, wherein the discrimination step comprises:

- the application to the signal of a low-pass filtering (48) on a time window of predetermined duration, and
- the comparison between the filtered signal (RSSI_BF) and the non-filtered signal (RSSI), to give a first signal type indicator.

3. The method of claim 1, wherein the discrimination step further comprises:

- the detection (50) of a peak level of the signal, and
- the comparison between the peak level of the signal (RSSI_PEAK) and the filtered signal (RSSI_BF), to give a second signal type indicator.

antenne

10

Attr
TX/RX

12

présélecteur

LNA

14

X

OL

FILTRE
canal

16

vers
démodulation

ADC

18

TX

unité de
traitement

20

**FIG_1**

début

analyse
long terme

22

demande
communication

28

mise à jour
résultat long
terme

24

analyse
court
terme

30

pause de
plusieurs
minutes

26

résultats
analyse
court terme

32

résultats
pondérés

34

sélection du canal
et établissement de
la communication

36

mise à jour des
paramètres de
pondération

38

**FIG_2**

## FIG_3

```
        ┌─────────┐
        │  canal  │──40
        │    i    │
        └─────────┘
             │
             ▼
   22,30  ┌──────────────┐
          │ échantillonner│──42
          │   le RSSI     │
          └──────────────┘
             │ RSSI
             ▼
      44 ─ ◇─────────┐ non
           │ est-ce  │──────────────────┐
           │ du bruit│                   │
           ◇─────────◇                   │
             │ oui                       │
             ▼                           ▼                    ▼
  46 ┌──────────────┐      48 ┌──────────┐      50 ┌──────────┐
     │  calcul et   │         │ filtrage │         │ détection│
     │  filtrage du │         │   BF     │         │  de pic  │
     │ bruit de fond│         └──────────┘         └──────────┘
     └──────────────┘
          │ Noise Floor        │ RSSI_BF           │ RSSI_Peak
          ▼                    ▼                   ▼
     ┌──────────────────────────────────────────────────────┐
     │ classifieur :                                         │
     │ - porteuse pure(niveau)                               │
     │ - modulation OOK(baud rate.codage etc...)             │
     │ - modulation FSK                               ──52    │
     │ - enveloppe de trame moyen                            │
     │ - duty cycle moyen                                    │
     │ - ......                                              │
     └──────────────────────────────────────────────────────┘
                         │
                         ▼
                  ┌─────────┐
                  │  canal  │──54
                  │   i+1   │
                  └─────────┘
```

RSSi

MAX

$M = \dfrac{MAX + MIN}{2}$

$M \pm \Delta dB$

MiN
Noise Floor

Si

t

FIG_4a

RSSi

MAX
MIN

S2

S3

Noise Floor

t

FIG_4b

RSSi

ch0   ch1   ch2   ch3   ch4   ch5   ch6   ch7   ch8   ch9

FIG_5

CANAL

FIG_6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1862195 A1
- US 6868288 B2
- US 20020045920 A1
- US 2008015655 A1, Bange